# EUROPÄISCHE PATENTANMELDUNG

(11) **EP 0 976 385 A2**
(43) Veröffentlichungstag der Anmeldung: **02.02.2000**
(21) Anmeldenummer: 99114132.6
(22) Anmeldetag: 19.07.1999
(51) Int. Cl.: A61K 7/13, D06P 1/02, D06P 1/32, D06P 1/34

(54) **Färbemittel für Haar und Fasern**

(30) Priorität: 25.07.1998 DE 19833545
(71) Anmelder: Wella Aktiengesellschaft, 64295 Darmstadt (DE)
(72) Erfinder: Sallwey, Anette, 63303 Dreieich (DE); Schmitt, Manfred, 64646 Heppenheim (DE); Dieckow, Andreas, 68309 Mannheim (DE)

(57) **Zusammenfassung**

Gegenstand der vorliegenden Erfindung ist ein Mittel zum Färben von Fasern, welches dadurch gekennzeichnet ist, daß es eine Kombination aus a) getrockneten und gemahlenen Indigoblättern und/oder Cassia Auriculata und/oder Kamille und b) bestimmten Lebensmittel- und Anfärbestoffen enthält.

## Beschreibung

Gegenstand der vorliegenden Erfindung ist ein Mittel zum Färben von Fasern auf der Basis von a) getrockneten und gemahlenen Indigoblättern und/oder Cassia Auriculata (Henna, neutral) und/oder Kamille und b) bestimmten Lebensmittel- und Anfärbefarbstoffen.

Die Nuancierungsmöglichkeiten von Formulierungen welche nur auf natürlichen Bestandteilen basieren sind äußerst begrenzt. Dies liegt an der geringen Auswahl der zu Verfügung stehenden Naturstoffe und an dem spezifischen Verhalten der Naturstoffe. So ergeben gemahlene Indigoblätter unmittelbar nach der Färbung ein grün-blaues Farbbild, das innerhalb von 72 Stunden nach der Behandlung in ein blau-rotes Farbbild umschlägt. Andere pflanzliche Rohstoffe, wie zum Beispiel Kamille, zeigen ein äußerst schwaches Farbergebnis. Lebensmittel- und Anfärbefarbstoffe sind zwar physiologisch unbedenklich, sie zeigen jedoch in der Regel ein sehr schwaches Farbergebnis und sind daher nur in Ausnahmefällen für Nuancierungen geeignet.

Es bestand daher die Aufgabe, ein Färbemittel auf der Basis von pflanzlichen Bestandteilen und Lebensmittel- und Anfärbefarbstoffen zur Verfügung zu stellen, das sowohl physiologisch unbedenklich ist als auch stabile und intensive Färbungen ermöglicht.

Hierzu wurde nunmehr gefunden, daß bei Verwendung einer Kombination aus a) getrockneten und gemahlenen Indigoblättern und/oder Cassia Auriculata und/oder Kamille und b) bestimmten Lebensmittel- und Anfärbefarbstoffen eine Verstärkung der Farbleistung und der Deckkraft sowie der Haltbarkeit erzielt werden kann. Weiterhin wurde überraschend festgestellt, daß bei der Verwendung einer Kombination aus Indigo und bestimmten Lebensmittel- und Anfärbefarbstoffen der bei Färbungen mit Indigo üblicherweise auftretende Farbumschlag von blau-grün nach blau-rot vermieden werden kann.

Gegenstand der vorliegenden Erfindung ist daher ein Mittel zum Färben von Fasern - insbesondere kereatinischen Fasern, wie zum Beispiel menschlichen Haaren-, welches dadurch gekennzeichnet ist, daß es eine Kombination aus a) getrockneten und gemahlenen Indigoblättern und/oder Cassia Auriculata und/oder Kamille und b) Lebensmittel- und Anfärbefarbstoffen enthält, wobei die Lebensmittel- und Anfärbefarbstoffe ausgewählt sind aus 6-Hydroxy-5-(4-sulfophenylazo)-2-naphthalinsulfonsäure-dinatriumsalz (Cl 15 985); 2- (1,3-Dioxo-2-indanyliden)-1,2-dihydrochinolindisulfonsäure-dinatriumsalz (Cl 47 005); 4- Hydroxy-3-(4-sulfo-1-naphthylazo)-1-naphthalinsulfonsäure-dinatriumsalz (Cl 14 720); 7-Hydroxy-8-(4-sulfo-1-naphthylazo)-1 ,3-naphthalindisulfonsäure-trinatriumsalz (Cl 16 255); 3-Hydroxy-4-(4-sulfo-1-naphthylazo)-2,7-naphthalindisulfonsäure-trinatriumsalz (Cl 16 185); 3',6'-Dihydroxy-2',4',5',7'-tetraiodospiro-[isobenzo-furan-1(3H),9'(9H)-xanthen]-3-on-Dinatriumsalz (Cl 45 430);1-(4'-Sulfonphenylazo)-2-hydroxy-6-sulfonaphthalin (Cl 15985); 1-(2'Hydroxy-4'sulfo-6'nitro)-naphthylazo-2-hydroxynaphthalin (Cl 15 700); 2,4-Dinitro-1-naphthol-7-sulfonsäure-Dinatriumsalz (Cl 10 316); 4,5-Dihydro-5-oxo-1-(4'-sulfophenyl)-4-[(4''-sulfo-phenyl)azo]-1H-pyrazol-3-carbonsäure-Trinatriumsalz (Cl 19 140); 3',6'-Dihydroxyspiro[isobenzofuran-1(3H),9'(9H)-xanthen]-3-on (Cl 45 350:1); 5-[2',4'-Dinitrophenyl)amino]-2-(phenylamino)-benzolsulfonsäure-Natriumsalz (Cl 10 385); 4-[(2',4'-Dihydroxyphenyl)azo]-benzolsulfonsäure-Natriumsalz (Cl 14 270); 3-Hydroxy-4-(3-methyl-5-oxo-1-phenyl-4,5-dihydro-1H-pyrazol-4-ylazo)-naphthalin-1-sulfonsäure-Natriumsalz,Chromkomplex; 4-[2'-Hydroxy-1'-naphthyl)azo]-benzolsulfonsäure-Natriumsalz (Cl 15 510); 4-[[3'-[(2'',4''-Dimethylphenyl)azo]-2',4'-dihydroxyphenyl]azo]-benzolsulfonsäure-Natriumsalz (Cl 20 170); 5-Amino-4-hydroxy-3-phenylazo-2,7-naphtalindisulfon-säure-Dinatriumsalz (Cl 17 200); 5-(Acetylamino)-4-hydroxy-3-[(2'-methyl-phenyl)azo]-2,7-naphthalin-disulfonsäure-Dinatriumsalz (Cl 18 065); 3,6-Bis-(diethylamino)-9-(2',4'-disulfophenyl)-xanthyliumhydroxid-Natriumsalz (Cl 45 100); 7-Hydroxy-8-[[4'-(phenylazo)phenyl]azo]-1,3-naphthalindisulfonsäure-Dinatriumsalz (Cl 27 290); 2',4',5',7'-Tetrabromo-3',6'-dihydroxyspiro-[isobenzo-furan-1(3H),9'(9H)-xanthen]-3-on-Dinatriumsalz (Cl 45 380); 2',4',5',7'-Tetrabromo-4,5,6,7-tetrachloro-3',6'-dihydroxy-spiro[isobenzofuran-1(3H),9'(9H)-xanthen]-3-on-Dinatriumsalz (Cl 45 410); 3',6'-Dihydroxy-4',5'-dijodospiro[isobenzofuran-1(3H),9'(9H)-xanthen]-3-on-Dinatriumsalz (Cl 45 425); N-Ethyl-N-[[4-[ethyl-[(3-sulfophenyl)methyl]amino]phenyl]-(sulfophenyl)methylen]-2,5-cyclohexadien-1-yliden]-3-sulfobenzolmethanaminiumhydroxid, inneres Salz, Dinatriumsalz (Cl 42 090); 2,2'-[(9,10-Dihydro-9,10-dioxo-1,4-anthracendiyl)-diimino]-bis-(5-methyl-benzolsulfonsäue)-Dinatriumsalz (Cl 61 570); N-[4-[[4'-(Dimethylamino)phenyl]-(2''-hydroxy-3'',6''-disulfo-1''-naphthyl)-methylen]-2,5-cyclohexadien-1-yliden]-N-methylmethan-aminiumhydroxid (Cl 44 090); N-[4-[[4'-Diethylamino)phenyl]-(2'',4''-disulfophenyl)-methylen]-2,5-cyclohexadien-1-yliden]-N-ethylethan-aminiumhydroxid-Natriumsalz (Cl 42 045); N-[4-[[4'-Diethylamino)phenyl)-(5''-hydroxy-2'',4''-disulfo-phenyl)-methylen]-2,5-cyclohexadien-1-yliden]-N-ethyl-ethanaminiumhydroxid-Calciumsalz (Cl 42 051); 1-Amino-4-(cyclohexylamino)-9,10-dihydro-9,10-dioxo-2-anthracensulfonsäure- Natriumsalz (Cl 62 045); 2-(1',3'-Dihydro-3'-oxo-5'-sulfo-2'H-indol-2'-yliden)-2,3-dihydro-3-oxo-1H-indol-5-sulfonsäure-Dinatriumsalz (Cl 73 015); 9-(2'-Carboxyphenyl)-3-[(2''-methylphenyl)amino]-6-[(2'''-methyl-4'''-sulfophenyl)amino)]-xanthylium-hydroxid-Natriumsalz (Cl 45 190); 3,3'-[Sulfonyl-bis(2-nitro-4,1-phenylen)imino]-bis-[6-phenylamino)-benzol-dinatriumsulfonat] (Cl 10 410); 4-Amino-5-hydroxy-3-[(4'-nitrophenyl)azo]-6-(phenylazo)-2,7-naphthalindisulfonsäure-Dinatriumsalz (Cl 20 470); 3-Hydroxy-4-[(2'-hydroxy-1'-naphthyl)azo]-7-nitro-1-naphthalinsulfonsäure-Natriumsalz (Cl 15 711); 3-[(2,4-Dimethyl-5-sulfophenyl)azo]-4-hydroxy-1-naphthalin-sulfonsäure (Cl 14 700); 6-[2,4-Dimethyl-6-soulfophenyl)azo]-5-hydroxy-1-naphthalinsulfonsäure-Dinatriumsalz (Cl 14 815); 6-Hydroxy-5-[(3-sulfophenyl)azo]-2-naphthalinsulfonsäure-Dinatriumsalz (Cl 15 980); 6-Hydroxy-5-[(2-methoxy-5-methyl-4-sulfophenyl)azo]-2-naphthalinsulfonsäure-Dinatriumsalz (Cl 16 035); Viktoriablau R (Cl 44 040); 1-(4-Sulfo-1-phenylazo)-4-amino-benzol-5-sulfonsäure (Cl 13 015); 1-(4-Sulfo-1-naphthylazo)-2-naphthol-3,6,8-trisulfonsäure (Cl 16 290); 5-Acetylamino-4-hydroxy-3-phenylazo-2,7-naphthalin-disulfonsäure-Dinatriumsalz (Cl 18 050); Acid Black 2 (Cl 50 420); 4-[(4-Aminophenyl)(4-imino-2,5-cyclohexadien-1-yliden)methyl]-2-methyl-aminobenzol-Hydrochlorid (Cl 42 510); 1,4-Diamino-9,10-anthrachinon; 3-[(4-cyclohexyl-2-methylphenyl)azo]-4-hydroxy-5-[[(4-methylphenyl)-sulfonyl]amino]-2,7-naphthalindisulfonsäure-Dinatriumsalz (Cl 18 130); Bis[2-[(4,5-dihydro-3-methyl-5-hydroxy-1-phenyl-1H-pyrazol-4-yl)azo]-benzoato(2), Chromkomplex (Cl 18 690); Bis[5-chloro-3-[(4,5-dihydro-3-methyl-5-hydroxy-1-phenyl-1H-pyrazol-4-yl)azol]-2-hydroxybenzol-sulfonato(3)-Dinatriumsalz (Cl 18 736); 4-[(4,5-Dihydro-3-methyl-5-hydroxy-4-(phenylazo)-1H-pyrazol-1-yl)]-benzosulfonsäure-Natriumsalz (Cl 18 820) und 2,5-Dichloro-4-[4,5-dihydro-3-methyl-5-hydroxy-4-[(sulfophenyl)azo]-1H-pyrazol-1-yl)]-benzosulfonsäure-Dinatriumsalz (Cl 18 965) oder Kombinationen dieser Farbstoffe.

Unter den vorstehend genannten Farbstoffen sind die folgenden besonders bevorzugt:
6-Hydroxy-5-(4-sulfophenylazo)-2-naphthalinsulfonsäure-dinatriumsalz (Cl 15 985); 2-(1,3-Dioxo-2-indanyliden)-1,2-dihydrochinolindisulfonsäure-dinatriumsalz (Cl 47 005); 4- Hydroxy-3-(4-sulfo-1-naphthylazo)-1-naphthalinsulfonsäure-dinatriumsalz (Cl 14 720); 7-Hydroxy-8-(4-sulfo-1-naphthylazo)-1,3-naphthalindisulfonsäure-trinatriumsalz (Cl 16 255); 3-Hydroxy-4-(4-sulfo-1-naphthylazo)-2,7-naphthalindisulfonsäure-trinatriumsalz (Cl 16 185) und 3',6'-Dihydroxy-2',4',5',7'-tetraiodospiro-[isobenzo-furan-1(3H),9'(9H)-xanthen]-3-on-Dinatriumsalz (Cl 45 430).

Die Gesamtmenge an Lebensmittel- und Anfärbefarbstoffen in dem Färbemittelgemisch beträgt etwa 0,01 bis 10 Gewichtsprozent, insbesondere 0,1 bis 8 Gewichtsprozent, wobei eine Menge von 0,1 bis 5 Gewichtsprozent besonders bevorzugt ist. Die Einsatzmenge der Lebensmittel- und Anfärbefarbstoffe in dem (gegebenenfalls mit Wasser vermischten) gebrauchsfertigem Färbemittel beträgt etwa 0,001 bis 5 Gewichtsprozent, vorzugsweise 0,002 bis 3,5 Gewichtsprozent.

Die Gesamtmenge an getrockneten und gemahlenen Indigoblättern und/oder Cassia Auriculata und/oder Kamille beträgt, bezogen auf die Gesamtmenge des Färbemittelgemisch, etwa 0,5 bis 99,5 Gewichtsprozent, insbesondere 1 bis 90 Gewichtsprozent.
Die Einsatzmenge an getrockneten und gemahlenen Indigoblättern und/oder Cassia Auriculata (Henna, neutral) und/oder Kamille beträgt, bezogen auf das (gegebenenfalls mit Wasser vermischte) gebrauchsfertige Färbemittel, etwa 0,05 bis 50 Gewichtsprozent vorzugsweise 0,1 bis 35 Gewichtsprozent.

Zusätzlich kann das erfindungsgemäße Färbemittel weitere für derartige Zusammensetzungen übliche Zusatzstoffe, beispielsweise Parfümöle; tierische Bestandteile wie Cochenille oder Lac Dye (kerria lacca); pflanzliche, nicht färbende Bestandteile, wie zum Beispiel Fichtennadeln, Akazienblätter, Lindenblätter, Birkenblätter, Weizen, Roggen, Gerste, Süßholz, Catechu oder Salbeikraut oder deren Kombinationen; Verdicker, wie zum Beispiel Cellulosen, Alginate, Xanthan-Gum, Galaktomannan-Polysaccharide, Polysaccharide und deren chemisch modifizierte Varianten, oder mineralische Verdicker wie Bentonit, Siliciumoxide und/oder Siliciumdioxide, Alkalistearate und/oder Erdalkalistearate, Stearate der 3. Hauptgruppe; Tenside und Emulgatoren; Antioxidantien wie Butylhydroxyanisol, Butyhydroxytoluol oder Tocopherol; Fruchtwachse; Chelatisierungsmittel oder Lösungs-vermittler wie Polyethylenglykole, beispielsweise Polyethylenglykol, enthalten.

Weiterhin kann das erfindungsgemäße Färbemittel zur Einstellung des gewünschten pH-Wertes Akalisierungsmittel, wie zum Beispiel Alkalihydroxide, Natriumcarbonat, Natriumhydrogencarbonat, Magnesiumcarbonat, Ammoniumcarbonat, Ammoniumhydrogencarbonat oder Natriumsilikate; oder Ansäuerungsmittel, wie zum Beispiel Zitronensäure, Weinsäure, Ammoniumchlorid oder Ammoniumsulfat, enthalten.

Zur Erzielung bestimmter Farbnuancen können dem erfindungsgemäßen Färbemittel weitere natürliche oder synthetische direktziehende Farbstoffe zugesetzt werden. Als natürliche direktziehende Farbstoffe können beispielsweise Hennablätter, Curcuma-Wurzeln, Faulbaumrinde, Olivenblätter, kanadische Blutwurzel,Gelbwurzel, Gelbholz, Rotholz, Rotsandelholz, Blauholz, Krappwurzel, Schwarzer Holunder oder Schwarze Apfelbeere oder deren Kombinationen verwendet werden; während als synthetische direktziehende Farbstoffe übliche physiologisch unbedenkliche Farbstoffe verwendet werden können.

Die natürlichen direktziehenden Farbstoffe können in dem erfindungsgemäßen Färbemittel, bezogen auf die Gesamtmenge des Färbemittelgemisches, in einer Menge von etwa 3 bis 95 Gewichtsprozent, vorzugsweise 5 bis 90 Gewichtsprozent, insbesondere 5 bis 50 Gewichtsprozent, eingesetzt werden, während die Einsatzmenge der synthetischen direktziehenden Farbstoffe, bezogen auf die Gesamtmenge des Färbemittelgemisches, etwa 0,01 bis 15, vorzugsweise 0,1 bis 12 Gewichtsprozent, insbesondere 0,1 bis 8 Gewichtsprozent, betragen kann.

Das erfindungsgemäße Färbemittel kann sowohl in Form eines Pulvers oder Granulates als auch in Form einer Emulsion, einer Dispersion oder einer Supension vorliegen.

Das erfindungsgemäße Färbemittel kann weiterhin sowohl in Form einer Einkomponenten-Zubereitung als auch in Form einer Mehrkomponenten-Zubereitung (insbesondere Zweikomponenten-Zubereitung) zur Anwendung kommen, wobei im Falle der Mehrkomponenten-Zubereitung jede der Komponenten sowohl in Form eines Pulvers oder Granulates als auch in Form einer Emulsion, einer Dispersion oder einer Supension vorliegen kann. So können beispielsweise die natürlichen Bestandteile in Pulverform getrennt von den übrigen Bestandteilen abgepackt werden und unmittelbar vor der Anwendung beziehungsweise vor dem Vermischen mit Wasser hinzugefügt werden. Ebenfalls ist es möglich, die erfindungsgemäßen Bestandteile a) und b) (insbesondere in fester Form) getrennt von den übrigen Bestandteilen abzupacken und unmittelbar vor der Anwendung beziehungsweise vor dem Vermischen mit Wasser hinzuzufügen.

Als Öle für die genannten emulsions-, dispersions- oder supensionsförmigen Färbemitteln eignen sich insbesondere flüssige Silikone oder Paraffine, pflanzliche oder tierische Öle, flüssige Fettsäuren, flüssige Fettsäureester, flüssige alkoxylierte Fettsäureester, flüssige Glyceride, flüssige alkoxylierte Glyceride, flüssige Fettalkohole, flüssige alkoxylierte Fettalkohole, insbesondere flüssige Fettalkoholethoxylate mit 2 bis 5 Ethylenoxideinheiten im Molekül, flüssige mehrwertige Alkohole, flüssige Nonylphenolether, oder flüssige Polypropylenglykole.

Als Wachse eignen sich insbesondere wachsförmige Silikone oder Paraffine, pflanzliche oder tierische Wachse, Fettsäuren, Fettsäureester, Glyceride, Monodiglyceride, Fettalkohole, alkoxylierte Fettalkohole, insbesondere, Polyethylenglykole oder Polypropylenglykole, Glyceride.

Die Öle und Wachse können, bezogen auf das gebrauchsfertige Mittel, in einer Gesamtmenge von etwa 0,1 bis 35 Gewichtsprozent enthalten sein.

Als Tenside und Emulgatoren können nichtionische, kationische, anionische oder amphotere oberflächenaktive Verbindungen und O/W- oder W/O-Emulgatoren, wie zum Beispiel Silikontenside, Alkyletherphosphate, Glyceridalkoxylate, Alkylsulfate, Alkalimetallsalze oder Erdalkalimetallsalze von Fettsäuren, Alkylethersulfate, Alkylsulfoacetate, oxethylierte Fettsäureester und Fettalkoholalkoxylate, verwendet werden.

Vor der Anwendung wird das Färbemittelgemisch mit kaltem oder warmen Wasser (T = 10 bis 100 °C) zu einem auftragefähigen Färbebrei vermischt, wobei dieses Vermischen in einer Schale oder durch Anschütteln in einer Auftrageflasche erfolgen kann. Es ist jedoch auch möglich, die Färbemittelformulierung direkt auf die vorher mit Wasser angefeuchtete Faser (zum Beispiel Haare) aufzutragen.

Das Mischverhältnis von Färbemittelgemisch zu Wasser beträgt vorzugsweise etwa 1: 1 bis 1: 100, wobei ein Verhältnis von 1: 2 bis 1: 50 besonders bevorzugt ist.

Im Falle der Mehrkomponentenzubereitung werden die einzelnen Komponenten vor der Anwendung (beziehungsweise vor dem Vermischen mit Wasser) miteinander vermischt, wobei beispielsweise das Mischungsverhältnis von natürlichen Bestandteilen zu den übrigen vorzugsweise etwa 10: 1 bis 1: 10 beträgt.

Der pH-Wert des gebrauchsfertigen erfindungsgemäßen Färbemittels liegt im sauren bis alkalischen Bereich (pH= 3,5 bis 12) und beträgt vorzugsweise 4,5 bis 10.

Das so erhaltene gebrauchsfertige Mittel zum Färben von Fasern ( 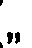 Färbebrei") wird auf die trockene oder handtuchfeuchte Faser gleichmäßig aufgetragen und nach einer Einwirkzeit von 5 bis 80 Minuten, vorzugsweise 15 bis 60 Minuten bei Raumtemperatur ( 20 bis 25 °C), beziehungsweise 10 bis 50 Minuten unter Wärmeeinwirkung (30 bis 50 °C), mit Wasser ausgespült und getrocknet.

Insbesondere im Falle von emulsionsförmigen, dispersionsförmigen oder supensionsförmigen Zubereitungen ist es jedoch auch möglich, das Färbemittel ohne vorheriges Vermisöhen mit Wasser direkt auf die mit Wasser angefeuchtete Faser aufzutragen.

Das erfindungsgemäße Färbemittel ermöglicht eine Verstärkung der Färbeleistung gegenüber üblichen Färbemitteln, wobei völlig unerwartet auch im alkalischen Bereich die beschriebene Farbstoffkombination aus Indigo und bestimmten Lebensmittel- und Anfärbefarbstoffen eine Färbung ergibt, die deutlich über den für die verwendeten Farbstoffe zu erwartenden Einzelergebnissen liegt. Aufgrund der hohen Intensität der mit der erfindungsgemäßen Farbstoffkombination erzielten Blaufärbungen sind nur geringe Mengen dieser Farbstoffkombination erforderlich. Die erfindungsgemäße Farbstoffkombination ermöglich zudem in Kombination mit anderen färbenden Pflanzenbestandteilen und/oder synthetischen direktziehenden Farbstoffen jede Nuancierung von blaurot bis rotbraun.

Obwohl die vorteilhaften Eigenschaften der Färbemittel (insbesondere bezüglich der guten physiologischen Verträglichkeit) bei der Färbung von Haaren besonders hervortreten, sind die erfindungsgemäßen Färbemittel ebenfalls hervorragend zur Färbung von anderen Fasern als Haaren, insbesondere Keratinfasern wie Seide oder Wolle, aber auch anderen Fasern wie zum Beipiel Viscose oder Baumwolle, geeignet.

Die nachfolgenden Beispiele sollen den Gegenstand näher erläutern, ohne diesen hierauf zu beschränken.

### Beispiele

### Beispiel 1: Haarfärbemittel

| **Nr.** | **Zusammensetzung** | **Menge** | **pH-Wert** |
|---|---|---|---|
| 1.1 | Indigo | 19,8 g | 9,9 |
| | 4- Hydroxy-3-(4-sulfo-1-naphthylazo)-1-naphthalin-sulfonsäure-Dinatriumsalz | 0,2 g | |
| | Natriumcarbonat | 6,0 g | |
| | Wasser (70 °C) | 74,0 g | |
| 1.2 | Indigo | 19,8 g | 6,5 |
| | 4- Hydroxy-3-(4-sulfo-1-naphthylazo)-1-naphthalin-sulfonsäure-Dinatriumsalz | 0,2 g | |
| | Wasser (70 °C) | 80,0 g | |

Die Färbemittelgemische 1.1 und 1.2 werden vor der Anwendung in einer Mischschale jeweils mit der angegebenen Menge an Wasser ( T = 70 °C) homogen vermischt und auf trockene gebleichte Haare aufgebracht. Nach einer Einwirkzeit von 30 Minuten bei 50 °C werden die Haare mit warmen Wasser ausgespült und anschließend getrocknet.

Mit dem vorstehend beschriebenen Färbemittel 1.1 wird direkt nach der Färbebehandlung ein dunkles Blauviolett erhalten, das sich im Verlauf der Zeit nur noch unwesentlich verändert; während das vorstehend beschriebene Färbemittel 1.2 direkt nach der Färbebehandlung ein leuchtendes Blaurot ergibt, das sich ebenfalls im Verlauf der Zeit nur noch unwesentlich verändert. Das mit den vorstehend beschriebenen Färbemitteln erzielte Färbeergebnis ist wesentlich intensiver als aufgrund der Addition der Ausfärbungen der Einzelkomponenten zu erwarten wäre.

### Beispiel 2: Haarfärbemittel

| **Nr.** | **Zusammensetzung** | **Menge** | **pH-Wert** |
|---|---|---|---|
| 2.1 | Cassia Auriculata | 19,8 g | 9,8 |
| | 2,4,5,7-Tetrajodofluorescein-Dinatriumsalz | 0,2 9 | |
| | Soda | 6,0 g | |
| | Wasser 70 °C | 74,0 g | |
| 2.2 | Cassia Auriculata | 19,8 g | 5,1 |
| | 2,4,5,7-Tetrajodofluorescein-Dinatriumsalz | 0,2 g | |
| | Wasser 70 °C | 80,0 g | |

Die Färbemittelgemische 2.1 und 2.2 werden vor der Anwendung in einer Mischschale jeweils mit der angegebenen Menge an Wasser ( T = 70 °C) homogen vermischt und auf trockene gebleichte Haare aufgebracht. Nach einer Einwirkzeit von 30 Minuten bei 50 °C werden die Haare mit warmen Wasser ausgespült und anschließend getrocknet.

Mit dem vorstehend beschriebenen Färbemittel 2.1 wird direkt nach der Färbebehandlung eine rosa Färbung erhalten, die sich im Verlauf der Zeit nur noch unwesentlich verändert; während das vorstehend beschriebene Färbemittel 2.2 direkt nach der Färbebehandlung ein Altrosaorange ergibt, das sich ebenfalls im Verlauf der Zeit nur noch unwesentlich verändert. Das mit den vorstehend beschriebenen Färbemitteln erzielte Färbeergebnis ist wesentlich intensiver als aufgrund der Addition der Ausfärbungen der Einzelkomponenten zu erwarten wäre.

### Beispiel 3: Haarfärbemittel

| **Nr.** | **Zusammensetzung** | **Menge** | **pH-Wert** |
|---|---|---|---|
| 3 | Kamille | 19,8 g | 9,9 |
| | 7-Hydroxy-8-(4-sulfo-1-naphthlazo)-1,3-naphthalin-disulfon-säure-Trinatriumsalz | 0,2 g | |
| | Soda | 6,0 g | |
| | Wasser 70 °C | 74,0 g | |

Das Färbemittelgemisch 3 wird vor der Anwendung in einer Mischschale mit der angegebenen Menge an Wasser ( T = 70 °C ) homogen vermischt und auf trockene gebleichte Haare aufgebracht. Nach einer Einwirkzeit von 30 Minuten bei 50 °C werden die Haare mit warmen Wasser ausgespült und anschließend getrocknet.

Es wird ein leuchtendes Orangerot erhalten, das wesentlich intensiver ist als aufgrund der Addition der Ausfärbungen der Einzelkomponenten zu erwarten wäre.

### Beispiel 4: Färbung von Fasern

Seide, Wolle, Baumwolle und Viskose werden jeweils mit einem Mittel gemäß den Beispielen 1 bis 3 in der dort beschriebenen Weise gefärbt, wobei die Trocknung der Faser in einem Trockenschrank bei 50 bis 60 °C erfolgt.

Es werden die gleichen Färbungen wie bei Haaren erhalten.

Alle in der vorliegenden Anmeldung genannten Prozentangaben bedeuten, soweit nicht anders angegeben, Gewichtsprozente.

## Patentansprüche

1. Mittel zum Färben von Fasern, dadurch gekennzeichnet, daß es eine Kombination aus a) getrockneten und gemahlenen Indigoblättern und/oder Cassia Auriculata und/oder Kamille und b) Lebensmittel- und Anfärbestoffen enthält, wobei die Lebensmittel- und Anfärbefarbstoffe ausgewählt sind aus 6-Hydroxy-5-(4-sulfophenylazo)-2-naphthalinsulfonsäure-dinatriumsalz; 2- (1,3-Dioxo-2-indanyliden)-1,2-dihydrochinolin-disutfonsäure-dinatriumsalz; 4- Hydroxy-3-(4-sulfo-1-naphthylazo)-1-naphthalinsulfonsäure-dinatriumsalz; 7-Hydroxy-8-(4-sulfo-1-naphthylazo)-1,3-naphthalindisulfonsäure-trinatriumsalz; 3-Hydroxy-4-(4-sulfo-1-naphthylazo)-2,7-naphthalindisulfonsäure-trinatriumsalz; 3',6'-Dihydroxy-2',4',5',7'-tetraiodospiro-[isobenzo-furan-1(3H), 9'(9H)-xanthen]-3-on-Dinatriumsalz;1-(4'-Sulfonphenylazo)-2-hydroxy-6-sulfo-naphthalin; 1-(2'Hydroxy-4'sulfo-6'nitro)-naphthylazo-2-hydroxynaphthalin; 2,4-Dinitro-1-naphthol-7-sulfonsäure-Dinatriumsalz; 4,5-Dihydro-5-oxo-1-(4'-sulfophenyl)-4-[(4''-sulfo-phenyl) azo]-1H-pyrazol-3-carbonsäure-Trinatriumsalz; 3',6'-Dihydroxyspiro [isobenzofuran-1(3H), 9'(9H)-xanthen]-3-on; 5-[2',4'-Dinitrophenyl)amino]-2-(phenylamino)-benzolsulfonsäure-Natriumsalz; 4-[(2',4'-Dihydroxyphenyl)azo]-benzolsulfonsäure-Natriumsalz; 3-Hydroxy-4-(3-methyl-5-oxo-1-phenyl-4,5-dihydro-1H-pyrazol-4-ylazo)-naphthalin-1-sulfonsäure-Natriumsalz, Chromkomplex; 4-[2'-Hydroxy-1'-naphthyl)azo]-benzolsulfonsäure-Natriumsalz; 4-[[3'-[(2'',4''-Dimethylphenyl)azo]-2',4'-dihydroxyphenyl]azo]-benzolsulfonsäure-Natriumsalz; 5-Amino-4-hydroxy-3-phenylazo-2,7-naphtalindisulfon-säure-Dinatriumsalz; 5-(Acetylamino)-4-hydroxy-3-[(2'-methylphenyl)azo]-2,7-naphthalin-disulfonsäure-Dinatriumsalz; 3,6-Bis-(diethylamino)-9-(2',4'-disulfophenyl)-xanthyliumhydroxid-Natriumsalz; 7-Hydroxy-8-[[4'-(phenylazo) phenyl]azo]-1,3-naphthalindisulfonsäure-Dinatriumsalz; 2',4',5',7'-Tetrabromo-3',6'-dihydroxyspiro-[isobenzo-furan-1(3H),9'(9H)-xanthen]-3-on-Dinatriumsalz; 2',4',5',7'-Tetrabromo-4,5,6,7-tetrachloro-3',6'-dihydroxy-spiro[isobenzofuran-1(3H),9'(9H)-xanthen]-3-on-Dinatriumsalz; 3',6'-Dihydroxy-4',5'-dijodospiro[isobenzofuran-1 (3H),9'(9H)-xanthen]-3-on-Dinatriumsalz; N-Ethyl-N-[[14-[ethyl-[(3-sulfophenyl)methyl]amino]phenyl]-(sulfophenyl)methylen]-2,5-cyclohexadien-1-yliden]-3-sulfobenzolmethanaminiumhydroxid, inneres Salz, Dinatriumsalz; 2,2'-[(9,10-Dihydro-9,10-dioxo-1,4-anthracendiyl)diimino]-bis-(5-methyl-benzolsulfonsäue)-Dinatriumsalz; N-[4-[[4'-(Dimethylamino)phenyl]-(2''-hydroxy-3'',6''-disulfo-1''-naphthyl)-methylen]-2,5-cyclohexadien-1-yliden]-N-methylmethan-aminiumhydroxid; N-[4-[[4'-Diethylamino)phenyl]-(2'',4''-disutfophenyl)-methylen]-2,5-cyclohexadien-1-yliden]-N-ethylethan- aminiumhydroxid-Natriumsalz; N-[4-[[4'-Diethylamino)phenyl]-(5''-hydroxy-2'',4''-disulfo-phenyl)-methylen]-2,5-cyctohexadien-1-yliden]-N-ethyl-ethanaminiumhydroxid-Calciumsatz; 1-Amino-4-(cyclohexylamino)-9,10-dihydro-9,10-dioxo-2-anthracensulfonsäure-Natriumsalz; 2-(1',3'-Dihydro-3'-oxo-5'-sulfo-2'H-indol-2'-yliden)-2,3-dihydro-3-oxo-1H-indol-5-sulfonsäure-Dinatriumsalz; 9-(2'-Carboxyphenyl)-3-[(2''-methylphenyl)amino)-6-[(2'''-methyl-4'''-sulfophenyl)amino)]-xanthylium-hydroxid-Natriumsalz; 3,3'-[Sulfonyl-bis(2-nitro-4,1-phenylen)imino]-bis-[6-phenylamino)-benzol-dinatriumsulfonat]; 4-Amino-5-hydroxy-3-[(4'-nitrophenyl)azo]-6-(phenylazo)-2,7-naphthalindisulfonsäure-Dinatriumsalz; 3-Hydroxy-4-[(2'-hydroxy-1'-naphthyl)azo]-7-nitro-1-naphthatinsulfonsäure-Natriumsalz; 3-[(2,4-Dimethyl-5-sulfophenyl)azo]-4-hydroxy-1-naphthalin-sulfonsäure; 6-[2,4-Dimethyl-6-soulfophenyl)azo]-5-hydroxy-1-naphthalinsulfonsäure-Dinatriumsalz; 6-Hydroxy-5-[(3-sulfophenyl)azo]-2-naphthalinsulfonsäure-Dinatriumsalz; 6-Hydroxy-5-[(2-methoxy-5-methyl-4-sulfophenyl)azo]-2-naphthalinsulfonsäure-Dinatriumsalz; Viktoriablau R; 1-(4-Sulfo-1-phenylazo)-4-amino-benzol-5-sulfonsäure; 1-(4-Sulfo-1-naphthylazo)-2-naphthol-3,6,8-trisulfonsäure; 5-Acetylamino-4-hydroxy-3-phenylazo-2,7-naphthalindisulfonsäure-Dinatriumsalz; Acid Black 2; 4-[(4-Aminophenyl)(4-imino-2,5-cyclohexadien-1-yliden)methyl]-2-methyl-aminobenzol-Hydrochlorid; 1,4-Diamino-9,10-anthrachinon; 3-[(4-cyclohexyl-2-methylphenyl)azo]-4-hydroxy-5-[[(4-methylphenyl)-sulfonyllamino]-2,7-naphthalindisulfonsäure-Dinatriumsalz; Bis[2-[(4,5-dihydro-3-methyl-5-hydroxy-1-phenyl-1H-pyrazol-4-yl)azo]-benzoato(2),Chromkomplex; Bis[5-chloro-3-[(4,5-dihydro-3-methyl-5-hydroxy-1-phenyl-1H-pyrazol-4-yl)azo]-2-hydroxybenzol-sulfonato(3)-Dinatriumsalz; 4-[(4,5-Dihydro-3-methyl-5-hydroxy-4-(phenylazo)-1H-pyrazol-1-yl)]-benzosulfonsäure-Natriumsalz und 2,5-Dichloro-4-[4,5-dihydro-3-methyl-5-hydroxy-4-[(sulfophenyl)azo]-1H-pyrazol-1-yl)]-benzosulfonsäure-Dinatriumsalz oder Kombinationen dieser Farbstoffe.

2. Mittel nach Anspruch 1, dadurch gekennzeichnet, daß der Lebensmittel- und Anfärbestoff ausgewählt ist aus 6-Hydroxy-5-(4-sulfophenylazo)-2-naphthalinsulfonsäure-dinatriumsalz; 2- (1,3-Dioxo-2-indanyliden)-1 ,2-dihydrochinolindisulfonsäure-dinatriumsalz; 4- Hydroxy-3-(4-sulfo-1-naphthylazo)-1-naphthalinsulfonsäure-dinatriumsalz; 7-Hydroxy-8-(4-sulfo-1-naphthylazo)-1,3-naphthalindisulfonsäure-trinatriumsalz; 3-Hydroxy-4-(4-sulfo-1-naphthylazo)-2,7-naphthalindisulfonsäure-trinatriumsalz und 3',6'-Dihydroxy-2',4',5',7'-tetraiodospiro-[isobenzo-furan-1(3H), 9'(9H)-xanthen]-3-on-Dinatriumsalz.

3. Mittel nach Anspruch 1 oder 2, dadurch gekennzeichnet, daß es die getrockneten und gemahlenen Indigoblätter und/oder Cassia Auriculata und/oder Kamille in einer Gesamtmenge von 0,5 bis 99,95 Gewichtsprozent enthält.

4. Mittel nach einem der Ansprüche 1 bis 3, dadurch gekennzeichnet, daß der Lebensmittel- und Anfärbestoff in einer Gesamtmenge von 0,01 bis 10 Gewichtsprozent enthalten ist.

5. Mittel nach einem der Ansprüche 1 bis 4, dadurch gekennzeichnet, daß es zusätzlich weitere natürliche oder synthetische direktziehende Farbstoffe enthält.

6. Mittel nach einem der Ansprüche 1 bis 5, dadurch gekennzeichnet, daß es in Form einer Einkomponentenzubereitung vorliegt.

7. Mittel nach einem der Ansprüche 1 bis 5, dadurch gekennzeichnet, daß es in Form einer Mehrkomponentenzubereitung vorliegt, wobei die natürlichen Bestandteile getrennt von den übrigen Bestandteilen abgepackt werden.

8. Verfahren zum Färben von Fasern, dadurch gekennzeichnet, daß die Faser mit Wasser angefeuchtet wird, sodann ein Färbemittel gemäß einem der Ansprüche 1 bis 7 auf die Faser aufgetragen wird und nach einer Einwirkungszeit von 5 bis 80 Minuten die Faser mit Wasser ausgespült und anschließend getrocknet wird.

9. Verfahren zum Färben von Fasern, dadurch gekennzeichnet, daß das Färbemittel gemäß einem der Ansprüche 1 bis 7 vor der Anwendung mit Wasser im Verhältnis 1 : 1 bis 1: 100 zu einem Färbebrei vermischt wird, sodann auf die Faser aufgetragen wird und nach einer Einwirkungszeit von 5 bis 80 Minuten die Faser mit Wasser ausgespült und anschließend getrocknet wird.
